# EUROPÄISCHE PATENTSCHRIFT

(11) **EP 2 413 112 B1**
(45) Veröffentlichungstag und Bekanntmachung des Hinweises auf die Patenterteilung: **21.06.2017**
(21) Anmeldenummer: 11173528.8
(22) Anmeldetag: 12.07.2011
(51) Int. Cl.: G01G 19/44, G01G 19/50, A61B 5/053

(54) **Personenwaage**
Personal weighing scale
Balance pour personnes

(30) Priorität: 28.07.2010 DE 102010038574
(43) Veröffentlichungstag der Anmeldung: 01.02.2012
(73) Patentinhaber: BSH Hausgeräte GmbH, 81739 München (DE)
(72) Erfinder: Weisbarth, Yvonne Janet, 82031 Grünwald (DE)

(56) Entgegenhaltungen:
- EP-A1- 1 304 552
- EP-A1- 1 705 467
- DE-A1- 10 125 176
- JP-A- 2000 060 935
- US-A1- 2002 134 589

## Beschreibung

Die vorliegende Erfindung betrifft eine Personenwaage mit einer Auftrittsfläche, auf welcher die zu wiegende Person stehen kann, gemäß dem Oberbegriff das Anspruchs 1.

Personenwaagen und insbesondere in jüngster Zeit auch Personenwaagen mit Messelektroden zur Messung körperspezifischer Eigenschaften, wie beispielsweise eines Körperfeltanteils, eines Muskelanteils oder einer Knochenmasse, freuen sich aufgrund eines zunehmenden Körperbewusstseins zunehmender Beliebtheit. Für eine exakte Erfassung des Gewichts ist dabei sowohl ein Untergrund unter der Personenwaage als auch ein richtiges Aufstellen der zu wiegenden Person auf der Personenwaage von entscheidender Bedeutung. Wird nämlich die Personenwaage falsch oder insbesondere auch ungleichmäßig belastet, so kann dies zu nicht unerheblichen Abweichungen hinsichtlich des gemessenen Gewichts führen, wodurch eine zuverlässige Gewichtsermittlung für die zu wiegende Person nicht mehr möglich ist. Die EP 1 304 552 A1 schlägt vor, die Genauigkeit und Reproduzierbarkeit der Meßergebnisse durch eine konvex gewölbte Oberschale zu verbessern. Auch die EP 1 705 467 A1 schlägt vor, eine korrekte Positionierung der zu wiegenden Person dadurch zu erreichen, dass die Oberfläche konvex ausgebildet ist. Die US 2002/134589 hingegen offenbart eine fußangepaßte Oberfläche zu Lösung des Problems.

Die vorliegende Erfindung beschäftigt sich daher mit dem Problem, für eine Personenwaagen der gattungsgemäßen Art eine verbesserte oder zumindest eine alternative Ausführungsform anzugeben, die sich insbesondere durch genaue Messergebnisse auszeichnet.

Dieses Problem wird erfindungsgemäß durch den Gegenstand des unabhängigen Anspruchs 1 gelöst. Vorteilhafte Ausführungsformen sind Gegenstand der abhängigen Ansprüche.

Die vorliegende Erfindung beruht auf dem allgemeinen Gedanken, eine Auftrittsfläche einer Personenwaage derart zu gestalten, dass die zu wiegende Person vorzugsweise nur in einer einzigen, nämlich der richtigen, Stellung auf der Personenwaage steht, wobei diese Stellung eine zuverlässige Messung zumindest des Gewichts erlaubt. Die Personenwaage weist dabei in bekannter Weise eine Auftrittsfläche auf, auf welche sich die zu wiegende Person zum Wiegen stellt, wobei diese Auftrittsfläche erfindungsgemäße eine mittige Erhebung aufweist, die als haptische Orientierung zum Auffinden der richtigen Standposition für die zu wiegende Person dient. Diese Wölbung bzw. haptisch wahrnehmbare Erhebung dirigiert somit die Person in eine vordefinierte Standposition, wobei diese Standposition so ausgewählt ist, dass sie zuverlässige Messergebnisse liefert. Selbstverständlich sollte die Personenwaage ähnlich wie bisherige Personenwaagen auch, auf einem festen und vorzugsweise ebenen Untergrund stehen, so dass durch den ebenen und festen Untergrund und die richtige Standposition durch die haptische Wölbung in der Auftrittsfläche der Personenwaage die Erfassung des Gewichts der zu wiegenden Person mit höchster Genaulgkeit erfolgen kann. Eine derartige mittige Erhebung bzw. haptisch wahrnehmbare Wölbung der Auftrittsfläche lässt sich sowohl fertigungstechnisch einfach als auch kostengünstig herstellen, wobei die Erhebung vorzugsweise derart geformt ist, dass auch Personen unterschiedlicher Größe, das heißt insbesondere Personen mit unterschiedlich großen Füßen, die für eine optimale Gewichtserfassung richtige Position leicht auffinden können. Die erfindungsgemäße Personenwaage ist somit nicht nur auf einen bestimmten Personenkreis, beispielsweise Erwachsene, beschränkt, sondern kann aufgrund der entsprechend ausgeformten Erhebung zur Gewichtserfassung unterschiedlichster Personen dienen. Für die vergleichsweise kostengünstig herzustellende mittige Erhebung kann somit die Genauigkeit der Gewichtsmessung deutlich verbessert werden, ohne dass hierfür teure und konstruktiv aufwändige Änderungen in einer Messelnrichtung selbst erforderlich wären.

Bei einer vorteilhaften Weiterbildung der erfindungsgemäßen Lösung, Ist die Erhebung an eine natürliche Wölbung einer menschlichen Fußsohle angepasst. Im günstigsten Fall stellt somit die Erhebung ein Negativ zur Wölbung der menschlichen Fußsohle dar, so dass die zu wiegende Person haptisch einfach die richtige Standposition auf der Personenwaage auffinden kann.

Bei einer weiteren vorteilhaften Ausführungsform der erfindungsgemäßen Personenwaage, ist diese mit mehreren Messelektroden zur Messung körperspezifischer Eigenschaften, wie beispielsweise eines Körperfettanteils, eines Muskelanteils oder einer Knochenmasse ausgestattet. Neben dem Gewicht kann eine derartig ausgebildete Personenwaage weitere gesundheitsrelevante Daten, wie insbesondere den Körperfettanteil, erfassen, wobei zur richtigen Erfassung ebenfalls eine richtige Standposition gewährleistet sein muss. Durch die mittige Erhebung wird die zu wiegende Person in die optimale Standposition dirigiert bzw. gezwungen, da ein anderes Stehen auf der erfindungsgemäßen Personenwaage unbequem ist bzw. von der zu wiegenden Person als unbequem empfunden und dadurch üblicherweise nicht eingenommen wird. Die Messelektroden sind selbst bei kostengünstigeren Varianten heute so genau, dass sie zumindest einen groben Überblick über die erfassten körperspezifischen Eigenschaften liefern können, so dass sich die zu wiegende Person mit der erfindungsgemäßen Personenwaage neben dem ermittelten Gewicht auch über weitere körperspezilische Eigenschaften informieren kann.

Erfindungsgemäß ist die Erhebung nachgiebig ausgebildet. Die erfindungsgemäß vorzugsweise mittig angeordnete Erhebung kann beispielsweise aus einer Silikon bzw. Gummischicht gebildet sein, so dass bei einem Falschstehen der zu wiegenden Person auf der erfindungsgemäßen Personenwaage das Falschstehen haptisch wahrnehmbar ist, wobei durch die nachgiebige Ausbildung der Erhebung dies nicht generell als schmerzhaft empfunden wird. Die zumindest geringfügige Nachgiebigkeit der Erhebung soll gleichzeitig dazu dienen, Personen mit Fußfehlstellungen oder Fußdeformationen die Verwendung der Personenwaage zu ermöglichen.

Weitere wichtige Merkmale und Vorteile der Erfindung ergeben sich aus den Unteransprüchen, aus den Zeichnungen und aus der zugehörigen Figurenbeschreibung anhand der Zeichnungen.

Bevorzugte Ausführungsbeispiele der Erfindung sind in den Zeichnungen dargestellt und wird in der nachfolgenden Beschreibung näher erläutert, wobei sich gleiche Bezugszeichen auf gleiche oder ähnliche oder funktional gleiche Bauteile beziehen.

Dabei zeigen, jeweils schematisch,
- Fig. 1: eine Draufsicht auf eine erfindungsgemäße Personenwaage,
- Fig. 2: eine Ansicht von der Seite auf die Personenwaage.

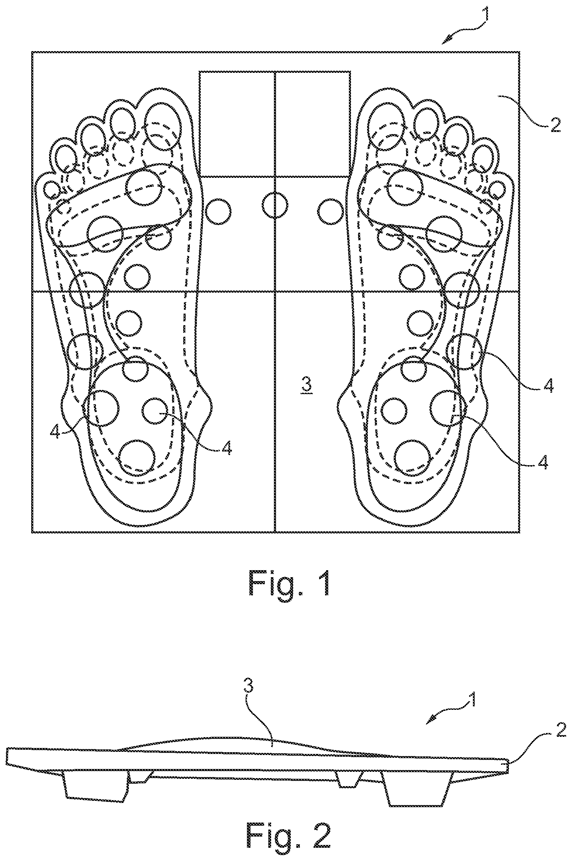

Entsprechend den Fig. 1 und 2, weist eine erfindungsgemäße Personenwaage 1 eine Auftrittsfläche 2 auf, auf welcher sich eine zu wiegende und nicht dargestellte Person zum Wiegen stellen kann. Erfindungsgemäß ist nun im Wesentlichen mittig der Auftrittsfläche 2 eine Erhebung 3 vorgesehen (vgl. Fig. 2) die als haptische Orientierung zum Auffinden der richtigen Standposition dient. Diese Erhebung 3 kann beispielsweise an eine natürliche Wölbung einer menschlichen Fußsohle angepasst sein, insbesondere ein Negativ zu dieser darstellen. Durch die Erhebung 3 gelingt es einer Person vergleichsweise einfach eine richtige Standposition auf der Personenwaage 1 aufzufinden, wodurch durch diese richtige Standposition eine zuverlässige Erfassung des Gewichts sowie anderer körperspezifischer Eigenschaften erleichtert wird. Zur Ermittlung anderer körperspezifischer Eigenschaften kann die Personenwaage 1 beispielsweise mehrere Messelektroden 4 aufweisen, mit welcher eine Messung eines Körperfettanteils, eines Muskelanteils oder einer Knochenmasse ermöglicht wird.

Vorzugsweise ist die Erhebung 3 zumindest geringfügig nachgiebig ausgebildet, insbesondere aus einem elastischen Werkstoff, wie beispielsweise Gummi oder Silikon, so dass ein falsches Stehen einer Person auf der Personenwaage 1 zwar haptisch fühlbar, nicht jedoch schmerzhaft ist. Die mittige Erhebung 3 ist vorzugsweise kreisförmig und mit einer konvexen Oberfläche ausgebildet, wobei die Auftrittsfläche 2 und/oder die Erhebung 3 beispielsweise mit einem rutschhemmenden Belag versehen sein können. Betrachtet man die Fig. 1, so kann man erkennen, dass die Messelektroden 4 einen Rand der Erhebung 3 nachbilden, so dass mit dem Auffinden der optimalen Standposition sich auch die Messelektroden 4 in einer optimalen Position zum Fuß befinden.

Die Erhebung 3 selbst kann beispielsweise einen Durchmesser von ca. 250 mm sowie eine Höhe von ca. 15 mm aufweisen. Außerhalb der Erhebung 3 ist die Auftrittsfläche 2 eben ausgebildet. Betrachtet man die Fig. 2, so kann man erkennen, dass die Erhebung 3 stufenlos in die diese umgebende Auftrittsfläche 2 ausläuft bzw. übergeht.

Mit der erfindungsgemäßen Personenwaage 1 wird eine Person beim Betreten der Auftrittsfläche 2 haptisch dazu angeregt, eine vorgegebene Standposition einzunehmen, wobei diese vordefinierte Standposition derart gewählt ist, dass hiermit besonders genaue Messungen, insbesondere des Gewichts, möglich sind. Zudem verbessert die Erhebung 3, die als gewöhnliche Wölbung ausgebildet sein kann, auch eine Standsicherheit für die zu wiegende Person, wodurch auch eine Unfallgefahr reduziert werden kann.

### Bezugszeichnliste

- 1: Personenwaage
- 2: Auftrittsfläche
- 3: Erhebung
- 4: Messelektrode

## Patentansprüche

1. Personenwaage (1) mit einer Auftrittsfläche (2), auf welche sich die zu wiegende Person stellt, wobei die Auftrittsfläche (2) eine mittige Erhebung (3) aufweist, die als haptische Orientierung zum Auffinden der richtigen Standposition dient, **dadurch gekennzeichnet, dass** die Erhebung (3) nachgiebig ausgebildet ist.

2. Personenwaage nach Anspruch 1, **dadurch gekennzeichnet, dass** die Erhebung (3) an eine natürliche Wölbung einer menschlichen Fußsohle angepasst ist.

3. Personenwaage nach Anspruch 1 oder 2, **dadurch gekennzeichnet, dass** die Personenwaage (1) mehrere Messelektroden (4) zur Messung körperspezifischer Eigenschaften, wie beispielsweise eines Körperfettanteils, eines Muskelanteils oder einer Knochenmasse, aufweist.

4. Personenwaage nach einem der Ansprüche 1 bis 3, **dadurch gekennzeichnet, dass** die mittige Erhebung (3) kreisförmig mit einer konvexen Oberfläche ausgebildet ist.

5. Personenwaage nach einem der Ansprüche 1 bis 4, **dadurch gekennzeichnet, dass** die Auftrittsfläche (2) zumindest außerhalb der Erhebung (3) mit einem rutschhemmenden Belag versehen ist.

6. Personenwaage nach einem der Ansprüche 1 bis 5, **dadurch gekennzeichnet, dass** die Erhebung (3) einen Durchmesser von ca. 250 mm aufweist.

7. Personenwaage nach einem der Ansprüche 1 bis 6, **dadurch gekennzeichnet, dass** die Erhebung (3) eine Höhe von ca. 15 mm aufweist.

8. Personenwaage nach einem der Ansprüche 1 bis 7, **dadurch gekennzeichnet, dass** die Erhebung (3) stufenlos in die diese umgebende Auftrittsfläche (2) übergeht.

## Claims

1. Personal weighing scales (1) with a stepping surface (2), on which the person to be weighed stands, wherein the stepping surface (2) has a central elevation (3), which acts as a haptic orientation for locating the correct standing position, **characterised in that** the elevation (3) is embodied to be soft.

2. Personal weighing scales according to claim 1, **characterised in that** the elevation (3) is adapted to a natural curvature of the sole of a human foot.

3. Personal weighing scales according to claim 1 or 2, **characterised in that** the personal weighing scales (1) have a plurality of measuring electrodes (4) for measuring body-specific properties, such as a body fat percentage, a muscle percentage or a bone percentage, for example.

4. Personal weighing scales according to one of claims 1 to 3, **characterised in that** the central elevation (3) is embodied to be circular with a convex surface.

5. Personal weighing scales according to one of claims 1 to 4, **characterised in that** the stepping surface (2) is provided, at least outside the elevation (3), with a slip-resistant coating.

6. Personal weighing scales according to one of claims 1 to 5, **characterised in that** the elevation (3) has a diameter of approx. 250 mm.

7. Personal weighing scales according to one of claims 1 to 6, **characterised in that** the elevation (3) has a height of approx. 15 mm.

8. Personal weighing scales according to one of claims 1 to 7, **characterised in that** the elevation (3) transitions steplessly into the stepping surface (2).

## Revendications

1. Pèse-personne (1) comprenant un plateau (2) sur lequel la personne à peser se place, le plateau (2) comportant un bossage central (3) servant d'orientation haptique pour trouver la bonne position debout, **caractérisé en ce que** le bossage (3) est réalisé de manière souple.

2. Pèse-personne selon la revendication 1, **caractérisé en ce que** le bossage (3) est adapté à une voûte naturelle d'une plante du pied de l'homme.

3. Pèse-personne selon la revendication 1 ou 2, **caractérisé en ce que** le pèse-personne (1) comporte plusieurs électrodes de mesure (4) servant à mesurer des caractéristiques spécifiques du corps, comme par exemple un taux de graisse corporelle, un taux de muscle ou une masse osseuse.

4. Pèse-personne selon l'une des revendications 1 à 3, **caractérisé en ce que** le bossage central (3) est de forme circulaire avec une surface convexe.

5. Pèse-personne selon l'une des revendications 1 à 4, **caractérisé en ce que** le plateau (2) est muni au moins en dehors du bossage (3) d'un revêtement antidérapant.

6. Pèse-personne selon l'une des revendications 1 à 5, **caractérisé en ce que** le bossage (3) a un diamètre d'environ 250 mm.

7. Pèse-personne selon l'une des revendications 1 à 6, **caractérisé en ce que** le bossage (3) a une hauteur d'environ 15 mm.

8. Pèse-personne selon l'une des revendications 1 à 7, **caractérisé en ce que** le bossage (3) se confond progressivement avec le plateau (2) l'entourant.
